# EUROPEAN PATENT SPECIFICATION

(11) **EP 0 313 535 B1**
(45) Date of publication and mention of the grant of the patent: **27.07.1994**
(21) Application number: 88850347.1
(22) Date of filing: 17.10.1988
(51) Int. Cl.: A61K 9/22, A61K 31/505

(54) **Oral formulation of buspirone and salts thereof**
Orales Präparat von Buspiron und seinen Salzen
Préparation orale de buspirone et ses sels

(30) Priority: 22.10.1987 SE 8704097
(43) Date of publication of application: 26.04.1989
(73) Proprietor: Bristol-Myers Squibb Company, New York, N.Y. 10154 (US)
(72) Inventor: Nicklasson, Alf Gunnar Martin, S-151 39 Södertälje (SE)
(74) Representative: Kinzebach, Werner, Dr.

(56) References cited:
- DE-A- 2 126 810
- US-A- 3 717 634
- US-A- 4 182 763
- US-A- 4 634 703
- US-A- 4 640 921
- Am. J. Med., vol. 80 (suppl. 3B), 1986, page 41, Metabolism and Disposition of Buspirone
- Controlled Drug Delivery: Fundamentals and Applications, 2nd Ed., Marcel Dekker Inc, USA, 1987, pages 23-24

## Description

The present invention relates to oral controlled/extended release dosage forms of buspirone and its salts, applying either matrix formulations, e.g. matrix tablets or multiparticular formulations, e.g. microcapsules or coated pellets, in order to obtain a drug delivery system of buspirone which will ensure a less frequent dosing during the day, e.g. a once daily dosing with a minimum of undesired side-effects. The formulation according to the invention will also provide for a higher amount of the dose to enter the general circulation compared to a conventional immediate release tablet, still with a minimum of side effects.

The physicochemical, pharmacokinetic and pharmacological properties of drugs and their products will often dictate how they should be used in a therapeutic situation. A drug characterized by a short biological half life must be administered in short dosage intervals to maintain the plasma concentration levels and hence the pharmacological action. This may greatly disrupt the patient compliance and it may cause underdosing between the dosage intervals. The ideal oral dosage form would be a once daily formulation to maintain the therapeutic drug levels in the body for 24 hours without the risk of any adverse reactions. The use of controlled/extended release dosage forms in drug therapy has increased in the last few years and the tendency toward once a day dosing has become common. Lists over controlled release products and their design can be rather extensive (L. Krowczynski, Extended Release Dosage Forms, CRC-Press Inc., USA, 1987, ISBN 0-8493-4307-0).

There are ways of avoiding the drawback of a short biological half life (rapid elimination) by technological means, namely due to the special dosage form which would ensure the desired drug release over an extended period of time and hence slowing of the drug absorption. Over the past two decades enormous progress has been made in developing controlled/extended release technologies for drug compounds. The design of controlled/extended release formulations and their technologies are well known in the art (L. Krowczynski, Extended Release Dosage Forms, CRC-Press Inc., USA, 1987, ISBN 0-8493-4307-0).

The purpose of production and administration of controlled/extended release dosage forms as well as the conditions limiting the possibilities of the preparations have been extensively outlined in the literature (L. Krowczynski, Extended Release Dosage Forms, CRC-Press Inc., USA, 1987, ISBN 0-8493-4307-0). Some important advantages of such delivery systems are:
- reduction of the frequency of dosage during the day (increased patient compliance)
- maintenance of therapeutic plasma drug levels for the period of time longer than the one indicated by the biological half life
- reduction of undesired adverse reactions, sometimes even toxic ones
- reduction of the total amount of drug required for a treatment by elimination of temporarily too high plasma concentrations.

DE-A-2 126 810 discloses a tablet for extended release of active ingredients which are contained in a threedimensional network which is insoluble in gastric juice. The threedimensional network consists of a plastics and/or waxy material in an amount of at most ten percent by weight of the tablet.

Buspirone, an azaspirondecanedione, with the structural formula 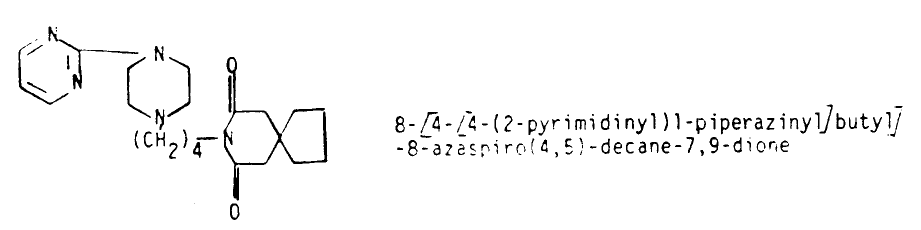
(disclosed in US 3 717 634) is a pharmaceutically active compound which has been found to be effective for the treatment of anxiety. However, buspirone shows a very high first pass metabolism and only about 4% of a therapeutic dose will unchanged reach the systemic circulation after oral administration (Mayol et.al, Clin.Pharmacol. Ther., 37, 210, 1985). Great interindividual variations regarding buspirone absorption have been observed showing a variation of the maximum plasma concentration by up to 10 fold (Gammans et.al, American J. Med., 80, Suppl. 3B, 41-51, 1986). Two metabolites have been identified, 5-OH-buspirone which shows no pharmacological activity and 1-(2-pyrimidinyl)-piperazine, i.e. 1-PP, which has been found to be about 20-25% as active as buspirone per se. The biological half life of buspirone itself is very short in man, e.g. 2-11 hours, whereas the much less active metabolite 1-PP shows a more slowly elimination (Mayol et. al, Clin. Pharmacol. Ther., 37, 210, 1985). These pharmacokinetic properties necessitates a rather frequent daily dosing which may have negative influences on the patient compliance. Since buspirone is rapidly absorbed after an oral dose, high plasma peak values occur short after drug administration which can be associated with the occurence of undesired adverse events which may occur rather instantly during the first days of treatment. This can also have serious impacts on patient compliance in terms of deliberated disrupture of the therapy.

Due to the fact that buspirone shows a very complicated pharmacokinetic behaviour, where the most striking phenomenon is an extensive first pass metabolism, the attempts to tackle the problem with buspirone oral absorption, e.g. to simplify the daily dosing without the risk of obtaining underdosing between each time of administration using a drug delivery system which will generate a minimum of undesired side effects have never been undertaken.

It is well known to the man in the art that an extensive or complex metabolic pattern of a drug will make the design of an oral controlled/extended release product very difficult, particularly when biological activity is wholly or partly due to a metabolite, as in the case of buspirone. It has thus been stated in the literature that differences in systemic availability due to metabolism during the absorptive process can be greater for controlled/extended release drug dosage forms than for immediate release drug products. Hence, it has been claimed that drugs which undergo extensive first pass clearance are unsuitable for oral controlled/extended release dosing (J.R. Robinson and V.H.L. Lee, Controlled Drug Delivery. Fundamentals and Applications, Marcel Dekker Inc., USA, 1987, ISBN 0-8247-7588-0). It has also been shown in the literature that metabolism of a drug, e.g. alprenolol, was more complete when it was administered in a controlled/extended release form than in conventional tablets (R. Johansson, C.G. Regårdh and J. Sjögren, Acta Pharm. Suec., 8,59 (1971)). Many other examples of similar matters have been reviewed in the literature (J.R. Robinson and V.H.L. Lee, Controlled Drug Delivery. Fundamentals and Applications, Marcel Dekker Inc., USA, 1987, ISBN 0-8247-7588-0).

Buspirone shows an extensive first pass metabolism with a ten-fold variation between subjects. Hence, due to this metabolic pattern in relation to the above mentioned examples and general statements from the literature regarding the suitability for such a drug to be used for a controlled/extended release dosage form is probably the reason why no prior attempts have ever been made to design an oral controlled/extended release product of buspirone.

The problem underlying the invention is to provide oral dosage forms of buspirone (single unit system and multi-particulate systems) having an increased bioavailability of the pharmacologically active component (i.e. unmetabolized buspirone) compared to conventional immediate released tablets and showing significantly fewer adverse effects (i.e. improved drug tolerability.

According to the invention it has now been found that it is possible to extend the oral absorption phase of buspirone without the formulation of too high plasma peak values by administering buspirone in certain controlled/extended release formulations. The plasma concentrations of buspirone were found to be extended and constant for a period of at least 18 hours after a 30 mg single dose and, during a 24 hour test period in vivo, a very high tolerability was observed compared to the administration of conventional buspirone tablets (3 x 10 mg). Furthermore, the mean relative extent of buspirone bioavailability was found to be increased by 5-6 times compared to the conventional dosage form of buspirone.

The present invention therefore relates to an oral formulation for controlled/extended release of buspirone or a salt thereof which is characterized in that the total in vitro dissolution time, determined according to the USP XXI paddle method at 50 or 100 rpm, is between 6 and 24 hours for at least 80 percent of the buspirone content.

The present invention provides an improved oral delivery system for buspirone as compared to conventional immediate release formulations. Different technologies can be applied for controlling and extending the release of buspirone (and hence its absorption in vivo) such as matrix formulations and multicompartment formulations (capsule or tablet containing pellets or microcapsules). This invention comprises various in vitro release time courses within each kind of technology by the application of various types and combinations of exipients and polymers. The present invention includes all kind of oral controlled release formulations known in the art which are able to show a total in vitro dissolution time between 6 and 24 hours for at least 80 percent of the drug content.

The in vitro dissolution time is measured by means of the USP XXI paddle method at 50 or 100 rpm. This method gives results in accordance with the flow through method using water at a flow rate of 16 ml/minute.

By either embedding the buspirone compound into a matrix formulation or incorporating it into a microcapsule formulation or both in order to control or extend the release, the following advantages are obtained compared to when conventional immediate release tablets are used:
- a slower in vivo absorption of buspirone and hence lower plasma peak values which hence reduce the occurrence of undesired side effects. This also means that the plasma peak values and the rate of increase of the plasma concentration for the less active metabolite 1-PP will be lower.
- prolonged and constant buspirone plasma concentrations over 24 hours which will avoid underdosing between dosage intervals.
- a significant increase of the relative extent of buspirone bioavailability (i.e. the therapeutically relevant component).
- much higher tolerability of the drug, i.e. much less side effects
- a once daily dosing of buspirone which together with the higher tolerability definitely will increase the patient compliance
- a lower cost for society since lower amount of buspirone will probably be needed due to the superbioavailability mentioned above.

Furthermore, the tablet formulations according to the present invention can be made smaller in size compared to the currently marketed buspirone tablet (BUSPAR®, Bristol-Myers, USA) even though they contain as much as three times the amount of buspirone. This will be another advantage regarding patient compliance since it must be much easier to swallow eg. a 6-8 mm round tablet once daily containing e.g. 15-30 mg buspirone compared to 1-2 tablets with a size of e.g. 6 x 10 mm 3 times daily (BUSPAR®, Bristol-Myers, USA).

Any salts of the active substance can be used, for instance acetate, benzenesulfonate, benzoate, bicarbonate, bitartrate, bromide, calcium edetate, camsylate, carbonate, chloride, citrate, dihydrochloride, edetate, edisylate, estolate, esylate, fumarate, gluceptate, gluconate, glutamate, glycollylarsanilate, hexylresorcinate, hydrabamine, hydrobromide, hydrochloride, hydroxynaphthoate, iodide, isethionate, lactate, lactobionate, malate, maleate, mandelate, mesylate, methylbromide, methylnitrate, methyl sulfate, mucate, napsylate, nitrate, pamoate, (embonate), pantothenate, phosphate/diphosphate, polygalacturonate, salicylate, stearate, subacetate, succinate, sulphate, tannate, tartrate, teoclate, triethiodide.

### Coating and matrix materials for obtaining controlled/extended release

The formation of microencapsulated material and matrix adjuvant is well known in the art.

As coating or matrix adjuvant any coating or matrix material can be used. The type of material will be chosen depending on the desired controlled release time-function i.e. whether it will be a dosage form having a 6, 12 or 24 hours release time in vitro. The choice of coating or matrix material will in either case be obvious to the man skilled in the art.

Coating and matrix materials to be used are for instance
Polymers:
synthetic polymers of polyvinyl type, e.g. poly vinylchloride,polyvinylacetate and copolymers thereof, polyvinylalcohol,polyvinylpyrrolidone.
Polyethylene type, e.g. polyethylene, polystyrene.
Polymers of acrylic acid or acrylic acid ester type, e.g. methylmetacrylate or copolymers of acrylic monomers.
Biopolymers or modified biopolymers of cellulose, e.g. ethylcellulose, cellulose acetate phtalate, cellulose acetate, hydroxy propyl cellulose, hydroxypropylmethyl cellulose, hydroxypropyl cellulose, methylcellulose, microcrystalline cellulose, Na-carboxymethyl cellulose.
Shellac
Gelatin
Fats, oils, higher fatty acids and higher alcohols e.g. aluminium monostearate, cetylalcohol, hydrogenated beef tallow, hydrogenated castor oil, 12-hydroxystearyl alcohol, glyceryl mono- or dipalmitate,
glyceryl mono- di-, or tristearate, myristyl alcohol, stearic acid, stearyl alcohol.
Polyethyleneglycols
Waxes e.g. bees wax, carnauba wax, Japan wax, paraffin, spermaceti, synthetic wax.
Sugars and sugar alcohols e.g. mannitol, sorbitol, sucrose, xylitol, glucose, maltose.

The polymers mentioned above can be used depending on the technique applied as coating agents, matrix adjuvants or pharmaceutical binders. Whether the polymer will be a matrix adjuvant or a pharmaceutical binder will be dependant on the amount of polymer in the formulation.

Any combinations of the above mentioned polymers, fats and waxes can be used for encapsulation purposes as well as for matrix formation, viz. different polymers can be mixed, a polymer can be mixed with a fat or wax etc.

The encapsulation of the drug can be achieved in the form of microcapsules, but the encapsulation is not restricted to the micro size.

The multiparticulate dosage forms, i.e. microcapsules or coated pellets as well as the matrix tablets useful for the present invention can be prepared by any of several acknowledged production processes including conventional granulation and tabletting of matrix tablets, pan coating, prilling, extrusion and spheronization, fluid bed processes, spray drying, spray chilling, coacervation and other processes.

### Microcapsules or coated pellets

Microcapsules or coated pellets are defined as a solid or liquid core enclosed in a coating. The coating may also be referred to as the wall or shell. Various types of microcapsule structures can be obtained depending on the manufacturing process e.g. mononuclear spherical, multinuclear spherical, multinuclear irregular, encapsulated mononuclear capsules, dual-walled microcapsules etc. Where no distinct coating and core region can be observed, the anologous terms are microparticles, microspheres, micromatrices, micro beads. The microcapsules or pellets usually have a particle size between 1 and 2000 microns.

The microcapsules or coated pellets of buspirone and its salts can be filled into empty hard gelatine capsules to an extent corresponding to the desired dose or they can be gently compressed into a tablet by using suitable tablet excipients.

Buspirone or a salt thereof could be mixed with a pharmaceutical binder to form micropellets which are then compressed into tablets.

The oral formulation of the invention could comprise micropellets which are then coated wiht a pharmaceutically acceptable coating adjuvant prior to being compressed into tablets.

The micropellets can also be filled into capsules.

The oral formulation of the invention could comprise microspheres which are then coated with a pharmaceutically acceptable coating adjuvant prior to being filled into capsules.

### Matrix formulations

Matrix formulations are defined as a drug embedded in insoluble excipients in order to achieve extended release by a continuous leaching of the drug from the inert matrix core. The release mechanism often follows the square root law of Higuchi (√t-law). This term also applies to a matrix built of hydrophilic substances which in contact with water form a gel of high viscosity.

### Preferred embodiments

A preferred embodiment of the present invention is obtained when buspirone hydrochloride is embedded in polyvinyl chloride and polyvinyl acetate and then compressed into a tablet formulation using magnesium stearate as lubricant (round tablet, 6-8 mm in diameter).

Other preferred embodiments are obtained when buspirone hydrochloride is embedded in polyvinyl chloride and ethyl cellulose by the addition of hydroxypropyl methyl cellulose, sodium carboxymethyl cellulose or paraffin. The material is then compressed into tablets using magnesium stearate as lubricant.

Still other preferred embodiments are when buspirone hydrochloride is mixed with a sugar derivative e.g. lactose and/or a cellulose derivative e.g. microcrystalline cellulose to obtain uncoated microspheres by means of extrusion and spheronization. The microspheres are then coated with e.g. ethyl cellulose using a suitable plasticizer e.g. triethyl citrate. The microcapsules can be filled into empty hard gelatine capsules.

Other preferred embodiments are when buspirone is suspended in a wax melt, e.g. carnauba wax, bees wax etc. and then spray chilled into microspheres. The spherical particles can then be coated with a fat or fatty acid, polyethylene glycol or a low melting wax by suspending the microspheres in the low melting excipient and then once again spray chill the slurry into microcapsules.

### Best mode of carrying out the invention

### Example 1

Buspirone hydrochloride was dry mixed with polyvinyl chloride. The powder mixture was then granulated with a solution of polyvinyl acetate in ethanol. After drying and milling, the granulation was compressed into 7 mm round tablets.

The buspirone controlled release tablets consist of (mg/tablet).

| | |
|---|---|
| Buspirone hydrochloride | 30 |
| Polyvinyl chloride | 120 |
| Polyvinyl acetate (C10-V7) | 11 |
| Magnesium stearate | 1.6 |

The in vitro dissolution was investigated in water by means of the USP XXI paddle method at different agitation conditions. The results are shown in Table 1.

**Table 1**

| Time (hours) | Range (% dissolved, n=6) | |
|---|---|---|
| | 50 rpm | 100 rpm |
| 1 | 34-35 | 32-39 |
| 3 | 57-58 | 53-57 |
| 5 | 71-73 | 67-71 |
| 8 | 84-88 | 81-87 |
| 12 | 92-96 | 90-95 |

This particular controlled release formulation of buspirone shows a 12 hour release time profile. As can be seen, a very uniform in vitro dissolution behaviour with a low interindividual variation between the six different tablet units are obtained. Hence, the design of the dosage form seems to be very robust in terms of in vitro dissolution characteristics.

The in vivo absorption and drug tolerability of this particulary formulation of buspirone were studied in six healthy subjects. As a reference, the same dose of conventional immediate release buspirone tablets was used, e.g. 3 x 10 mg, BUSPAR®, Bristol-Myers, USA.

Table 2 shows typical plasma concentration versus time courses of buspirone for the two products tested in two different subjects. As can be seen, significant different plasma profiles are obtained with the two buspirone products. The invented controlled/extended release dosage form of buspirone shows much lower plasma concentration peaks, a slower rate of absorption and much more extended plasma concentration versus time courses compared to the conventional tablet formulation of buspirone. Buspirone can be detected in plasma 24 hours after administration of the invented formulation whereas no detectable buspirone concentrations can be found 4 to 8 hours after dosing of the conventional immediate release tablets.

**Table 2**

| Time (h) | Invented product (ng/ml) | | Conventional product (ng/ml) | |
|---|---|---|---|---|
| | Subj. 1 | Subj. 4 | Subj. 1 | Subj. 4 |
| 0.5 | 1.87 | 1.41 | 2.28 | 4.04 |
| 1 | 2.33 | 1.80 | 0.89 | 2.96 |
| 2 | 2.33 | 2.12 | 0.58 | 0.85 |
| 4 | 1.44 | 1.72 | 0.40* | ND |
| 6 | 2.20 | 2.02 | 0.08* | ND |
| 8 | 1.56 | 2.13 | 0.08* | ND |
| 12 | 1.66 | 1.92 | ND | ND |
| 18 | 0.75 | 0.26* | ND | ND |
| 24 | 0.75 | 0.24* | ND | ND |
| ND = not detected | | | | |

| | | | | |
|---|---|---|---|---|
| * = below the lowest standard calibrator used in the assay (0.5 ng/ml) | | | | |

Table 3 shows the relative extent of buspirone bioavailability for the invented controlled/extended release tablet compared to the conventional formulation. Dose = 30 mg.

**Table 3**

| Subject No | Rel. extent of buspirone bioavailability for the invented formulation |
|---|---|
| 1 | 16.8 |
| 2 | 11.9 |
| 3 | 1.2 |
| 4 | 4.7 |
| 5 | 1.4 |
| 6 | 0.4 |

As can be seen, in 5 out of 6 subjects, a significant increase in buspirone bioavailability is obtained if the rate of drug release from the dosage form is decreased by means of the invented product design. Hence, the buspirone has become superbioavailable by means of the invented controlled/extended release dosage form. Even if significantly higher amounts of buspirone will enter the general circulation during a 24 hour time period compared to after administration of conventional tablets, an improved drug tolerability is obtained by administering buspirone in a controlled/extended release dosage form according to the present invention.

Table 4 shows the observed side effects in each subject at a single dose of 30 mg.

**Table 4**

| Subject No | Adverse experience (severity)* | |
|---|---|---|
| | Invented product according to Example 1 | Conventional product |
| 1 | Frontal headache (2) | None |
| 2 | None | Dizziness & light-headed (2) |
| 3 | None | Dizziness & paraesthesia (1) |
| 4 | None | Dizziness (2) |
| 5 | None | Dizziness & paraesthesia in both hands, dizziness postural in nature. Nausea on sitting up (3) |
| 6 | None | None |

| | | |
|---|---|---|
| * | | |
| (1) = mild, | | |
| (2) = moderate, | | |
| (3) = severe | | |

It is very obvious that the present invention will be a great impr ment in the therapeutic application of buspirone in terms of drug tolerability.

As mentioned above, the extent of buspirone bioavailability was significantly improved. However, another positive effect caused by the present invention of buspirone is a decrease of the bioavailability of the less active metabolite 1-PP. The present invention creates much lower plasma peak values of 1-PP and a slower rate of the increase of its plasma concentrations which may also be a contributing factor for the good tolerability obtained after administration of the present buspirone invention. Table 5 summarizes the relative extent of 1-PP bioavailability after administration of the buspirone controlled/extended release tablet according to Example 1, compared to a conventional tablet formulation.

**Table 5**

| Subject No | Rel. extent of 1-PP bioavailability for invented product |
|---|---|
| 1 | 0.7 |
| 2 | 0.5 |
| 3 | 0.4 |
| 4 | 0.9 |
| 5 | 0.7 |
| 6 | 1.1 |

### Example 2

Buspirone hydrochloride was dry mixed with polyvinyl chloride. The powder mixture was then granulated with polyvinyl acetate dissolved in ethanol. After drying and milling, the granulation was compressed into round matrix tablets with a diameter of 8 mm.

The buspirone controlled release matrix tablets consist of (mg/tablet)

| | |
|---|---|
| Buspirone hydrochloride | 30 |
| Polyvinyl chloride | 160 |
| Polyvinyl acetate | 20 |
| Magnesium stearate | 2.1 |

The in vitro dissolution was determined in water by means of the USP XXI Paddle method at various agitation conditions, see Table 6.

**Table 6**

| Time (hours) | Range (% dissolved, n=6) | |
|---|---|---|
| | 50 rpm | 100 rpm |
| 1 | 19-20 | 22-23 |
| 3 | 35-36 | 36-39 |
| 5 | 44-46 | 48-50 |
| 8 | 56-58 | missing |
| 12 | 68-71 | 68-71 |
| 15 | 75-78 | 81-85 |
| 20 | 83-85 | 88-93 |
| 24 | > 90 | > 90 |

This particularly controlled release formulation of buspirone shows a 24 hour release time course. As can be seen in Table 6, this formulation of buspirone shows a very uniform in vitro dissolution behaviour with a low interindividual variation between the six different tablet units which is in good agreement with the data shown in Example 1 for a 12 hours controlled release matrix tablet. Hence, as was mentioned before, the principle of embedding buspirone into a polyvinyl chloride/polyvinyl acetate matrix will produce a robust and reliable drug delivery system which is unaffected by the hydrodynamic intensity.

The in vivo absorption and drug tolerability were also investigated for this 24 hour release time formulation of buspirone. Six healthy subjects participated in the study using conventional immediate release tablets (3 x 10 mg), BUSPAR®, Bristol-Myers, USA as reference.

Table 7 shows typical plasma concentration versus time courses of buspirone in two subjects after administration of the invented controlled release formulation. As a reference, plasma concentration versus time courses are also shown after administration of conventional buspirone tablets (BUSPAR®, Bristol Myers, USA) at a single dose of 30 mg. As can be seen, highly different plasma profiles were obtained. Once again, the invented product of buspirone shows much lower plasma concentration peaks, a slower rate of absorption and much more extended plasma concentration versus time courses compared to the conventional tablet formulation. Thus, even if the in vitro dissolution time is increased from 12 hours to 24 hours (cf. Example 1), a sufficient absorption of buspirone is achieved. After 24 hours, significant plasma concentration levels are found in the two subjects after administration of the invented buspirone formulation, whereas no detectable concentrations or very low ones were obtained for the conventional dosage form 4 to 8 hours after dosing.

**Table 7**

| Time (h) | Invented product (ng/ml) | | Conventional product (ng/ml) | |
|---|---|---|---|---|
| | Subj. 4 | Subj. 5 | Subj. 4 | Subj. 5 |
| 0.5 | 1.01 | 0.26* | 4.07 | 6.31 |
| 1 | 2.43 | 0.41* | 2.96 | 3.28 |
| 2 | 2.05 | 0.99 | 0.85 | 2.99 |
| 4 | 1.98 | 1.11 | ND | 1.49 |
| 6 | 2.31 | 0.97 | ND | 0.47* |
| 8 | 0.04* | 0.86 | ND | 0.22* |
| 12 | 0.87 | 1.03 | ND | 0.05* |
| 18 | 1.03 | 0.72 | ND | 0.09* |
| 24 | 1.80 | 0.74 | ND | 0.05* |
| ND = not detected | | | | |

| | | | | |
|---|---|---|---|---|
| * = below the lowest standard calibrator used in the assay (0.5 ng/ml). | | | | |

Table 8 shows the relative extent of buspirone bioavailability for the invented 24 hour release formulation of buspirone compared to the conventional tablet formulation (BUSPAR®, 3 x 10 mg, Bristol-Myers, USA).

**Table 8**

| Subject No | Rel. extent of buspirone bioavailability for the invented formulation |
|---|---|
| 1 | Missing data |
| 2 | 8.2 |
| 3 | 1.3 |
| 4 | 6.3 |
| 5 | 1.7 |
| 6 | 0.4 |

An increase of buspirone bioavailability can be seen for the invented 24 hour controlled/extended release formulation which is in agreement with the absorption data found for the 12 hour release formulation in Example 1. The plasma concentrations in subject no 1 were found to be detectable but below the lowest standard calibrator used in the assay, i.e. 0.5 ng/ml. This is the reason why the relative extent of buspirone bioavailability has not been given in Table 8. However, if the raw data are used in a calculation, a value of 2.8 is found for subject no 1. It is only in subject No 6 where a lower bioavailability can be seen which also is in agreement with the data shown in Example 1 (the same healthy subjects were used in both studies).

The adverse experiences obtained for the different buspirone formulations after a single dose of 30 mg are reported in Table 9.

**Table 9**

| Subject No | Adverse experience (severity)* | |
|---|---|---|
| | Invented product according to Example 2 | Conventional product |
| 1 | None | None |
| 2 | None | Dizziness & light-headed (2) |
| 3 | None | Dizziness & paraesthesia (1) |
| 4 | None | Dizziness (2) |
| 5 | None | Dizziness & paraesthesia in both hands (fingertips only), dizziness postural in nature. Nausea on sitting up (3) |
| 6 | None | None |

| | | |
|---|---|---|
| * | | |
| (1) = mild | | |
| (2) = moderate | | |
| (3) = severe | | |

Once again, it is very obvious that the present invention will be great improvement in the therapeutic application of buspirone in ter of drug tolerability.

Table 10 shows the corresponding bioavailability data for the 1-PP metabolite.

**Table 10**

| Subject No | Rel. extent of 1-PP bioavailability for the invented product |
|---|---|
| 1 | 0.44 |
| 2 | 0.16 |
| 3 | 0.16 |
| 4 | 0.70 |
| 5 | 0.78 |
| 6 | 1.02 |

The results in Examples 1 and 2 have shown that buspirone controlled/extended release formulations can be designed either as 12 hours or 24 hours release products. For both kinds of products, significantly higher extent of buspirone bioavailabilities and a very low frequency of side effects were obtained compared to a single dose administration of a conventional tablet formulation. Hence, the most optimum use of buspirone in a therapeutic situation would be the application of a controlled/extended release product. Both a 12 and a 24 hours release formulation can be used for obtaining an extended plasma concentration versus time course suitable for a once daily dosing. The present invention will provide for a higher amount of the dose to enter the general circulation but with the occurrence of a much lower frequency of side effects. The invention will also guarantee that no underdosing will occur between dosage as will probably be the case for a conventional formulation of buspirone. These advantages of the present invention compared to the current conventional market formulation (tablets) of buspirone will definitely make therapy easier for the patients and hence, an increase in patient complience is to be expected.

### Example 3

Buspirone hydrochloride was dry mixed with polyvinyl chloride. The powder mixture was granulated with a solution of polyvinyl acetate dissolved in ethanol. After drying and milling, the granulation was compressed into round matrix tablets with a diameter of 6 mm.

The buspirone controlled/extended release tablets consist of (mg/tablet)

| | |
|---|---|
| Buspirone hydrochloride | 15 |
| Polyvinyl chloride | 80 |
| Polyvinyl acetate | 7.3 |
| Magnesium stearate | 1.0 |

The in vitro dissolution data are presented in Table 11 using the USP XXI paddle method at 50 rpm (water).

**Table 11**

| Time (hours) | Range (% dissolved, n=6) |
|---|---|
| 3 | 50-57 |
| 6 | 67-77 |
| 12 | 85-96 |

The present formulation relates to a 12 hours release product containing 15 mg buspirone hydrochloride per tablet.

### Example 4

Buspirone hydrochloride was dry mixed with polyvinyl chloride and sodium carboxymethyl cellulose. The powder mixture was granulated with a solution of ethyl cellulose 10 mPas (10 cps) dissolved in ethanol. After drying and milling, the granulation was compressed into tablets using magnesium stearate as lubricant (8 mm round tablets).

The buspirone controlled/extended release tablets consist of (mg/tablet)

| | |
|---|---|
| Buspirone hydrochloride | 30 |
| Polyvinyl chloride | 145 |
| Ethyl cellulose 10 mPas (10 cps) | 10 |
| Sodium-carboxymethyl cellulose | 15 |
| Magnesium stearate | 1.95 |

Table 12 shows the in vitro dissolution characteristics for this particulary controlled release formulation of buspirone using the USP XXI paddle method at 50 rpm (water).

**Table 12**

| Time (hours) | Range (% dissolved, n=3) |
|---|---|
| 1 | 26-32 |
| 2 | 47-52 |
| 6 | 85-94 |

As can be seen, the above formulation shows a 6 hours release time course of buspirone in vitro.

### Example 5

Buspirone hydrochloride was dry mixed with lactose and microcrystalline cellulose. Water was added and the wet powder mass was extruded in a NICA-extruder using a 1 mm screen. The extrudates were spheronized on a marumerizer plate into microspheres to a size range of 0.8-1.4 mm. The wet microspheres were dried and then overcoated with ethyl cellulose 10 mPas (10 cps) in a fluid bed apparatus. The ethyl cellulose was dissolved in a 6/4 mixture of methylene chloride and ethanol. Triethyl citrate was used as plasticizer in the polymer film.

The buspirone controlled/extended release microcapsules consist of the following. A 30 mg dosage strength (i.e. a hard gelatine capsule) is used in this particulary Example. However, lower as well as higher dosage strengths can easily be obtained by decreasing or increasing the amount of microcapsules administered e.g. in a hard gelatine capsule.

| | mg/capsule |
|---|---|
| Buspirone hydrochloride | 30 |
| Lactose | 180 |
| Microcrystalline cellulose | 120 |
| Ethyl cellulose 10 mPas (10 cps) | 20 |
| Triethyl citrate | 2 |

Table 13 shows the in vitro dissolution results for the buspirone controlled/extended release microcapsules using the USP XXI paddle method at 50 rpm (water).

**Table 13**

| Time (hours) | Range (% dissolved, n=3) |
|---|---|
| 1 | 20-22 |
| 3 | 51-53 |
| 6 | 72-74 |
| 12 | 88-90 |

As can be seen, buspirone can be formulated into a multiparticular controlled/extended release formulation showing, as in this Example, a 12 hour release time course in vitro.

## Claims (Claims for the following Contracting State(s): AT, BE, CH, DE, FR, GB, IT, LI, LU, NL, SE)

1. An oral formulation for controlled/extended release of buspirone or a salt thereof **characterized in that** the total in vitro dissolution time, determined according to the USP XXI paddle method at 50 or 100 rpm, is between 6 and 24 hours for at least 80 percent of the buspirone content.

2. A formulation according to claim 1 **characterized in that** it contains from 1 to 80 percent weight/weight of buspirone or a salt thereof.

3. A formulation according to claim 1 or 2 in the form of a matrix tablet or multicompartment formulation, in particular a capsule or tablet containing pellets or microcapsules.

4. A formulation according to any of claims 1 to 3, **characterized in** that it consists of buspirone or a salt thereof mixed and/or coated with an adjuvant selected from the group consisting of a synthetic polymer of the polyvinyl type, the polyethylene type or the cellulose type, fats, waxes and sugars or combinations thereof.

5. A formulation according to any of claims 1 to 4 in the form of a tablet containing buspirone hydrochloride embedded in polyvinyl chloride and polyvinyl acetate and magnesium stearate as lubricant.

6. A formulation according to any of claims 1 to 4 in the form of a tablet containing buspirone hydrochloride embedded in polyvinyl chloride and ethyl cellulose and additionally hydroxypropyl methyl cellulose, sodium carboxymethyl cellulose or paraffin, as well as magnesium stearate as lubricant.

7. A formulation according to any of claims 1 to 3 in the form of a hard gelatin capsule filled with microcapsules or coated pellets, or coated or uncoated micropellets containing buspirone or a salt thereof.

8. A formulation according to claim 7 in the form of a hard gelatin capsule filled with coated microspheres containing buspirone hydrochloride mixed with a sugar and/or cellulose derivative.

9. A formulation according to any of claims 1 to 3 in the form of a tablet comprising microcapsules or coated or uncoated micropellets.

10. A process for the preparation of an oral formulation, according to anyone of claims 1 to 9, characterized in that buspirone or a salt thereof is incorporated into a carrier which releases buspirone or a salt thereof in a total in vitro dissolution time , determined according to the USP XXI paddle method at 50 or 100 rpm, between 6 and 24 hours for at least 80 per cent of the buspirone content.

11. The process according to claim 10 **characterized in** that buspirone or a salt thereof is mixed and/or coated with an adjuvant selected from the group consisting of a synthetic polymer of the polyvinyl type, the polyethylene type or the cellulose type, fats, waxes and sugars or combinations thereof and then either compressed into tablets or processed into microcapsules/pellets which are either filled into hard gelatin capsules or compressed into tablets.

## Claims (Claims for the following Contracting State(s): ES, GR)

1. A process for the preparation of an oral formulation for controlled/extended release of buspirone or a salt thereof having a total in vitro dissolution time of between 6 and 24 hours for at least 80 percent of the buspirone content, characterized in that buspirone or a salt thereof is incorporated into a carrier which releases buspirone or a salt thereof in said in vitro dissolution time.

2. A process according to claim 1 which comprises the preparation of a formulation containing from 1 to 80 percent weight/weight of buspirone or a salt thereof.

3. A process according to claim 1 or 2 which comprises the preparation of a matrix tablet or multicompartment formulation, in particular a capsule or tablet containing pellets or microcapsules.

4. A process according to any of claims 1 to 3, which comprises the preparation of a formulation consisting of buspirone or a salt thereof mixed and/or coated with an adjuvant selected from the group consisting of a synthetic polymer of the polyvinyl type, the polyethylene type or the cellulose type, fats, waxes and sugars or combinations thereof and then either compressed into tablets or processed into microcapsules/pellets which are either filled into hard gelatin capsules or compressed into tablets.

5. A process according to any of claims 1 to 4 which comprises the preparation of a tablet containing buspirone hydrochloride embedded in polyvinyl chloride and polyvinyl acetate and with magnesium stearate as lubricant.

6. A process according to any of claims 1 to 4 which comprises the preparation of a tablet containing buspirone hydrochloride embedded in polyvinyl chloride and ethyl cellulose and additionally hydroxypropyl methyl cellulose, sodium carboxymethyl cellulose or paraffin as well as magnesium stearate as lubricant.

7. A process according to anyone of claims 1 to 3 which comprises the preparation of a hard gelatin capsule filled with microcapsules or coated pellets or coated or uncoated micropellets containing buspirone or a salt thereof.

8. A process according to claim 7 which comprises the preparation of a hard gelatin capsule containing buspirone hydrochloride mixed with a sugar and/or cellulose derivative to form uncoated microspheres which are then overcoated and filled into capsules.

9. A process according to anyone of claims 1 to 3 which comprises the preparation of a tablet comprising microcapsules or coated or uncoated micropellets.

10. A process according to claim 4, wherein the formulation is either compressed into tablets or processed into microcapsules/pellets which are either filled into hard gelatin capsules or compressed into tablets.

## Patentansprüche (Patentansprüche für folgende(n) Vertragsstaat(en): AT, BE, CH, DE, FR, GB, IT, LI, LU, NL, SE)

1. Orale Formulierung zur kontrollierten/verzögerten Freisetzung von Buspiron oder einem Salz davon,
**dadurch gekennzeichnet,** daß die in vitro-Gesamtauflösungszeit, die gemäß der USP XXI Paddle-Methode bei 50 bis 100 UpM bestimmt wird, für wenigstens 80 % des Buspirongehaltes zwischen 6 bis 24 Stunden beträgt.

2. Formulierung gemäß Anspruch 1,
**dadurch gekennzeichnet**, daß sie 1 bis 80 % Gew./Gew. Buspiron oder eines Salzes davon enthält.

3. Formulierung gemäß Anspruch 1 oder 2 in Form einer Matrixtablette oder einer Formulierung mit mehreren Kompartimenten, insbesondere eine Kapsel oder Tablette, welche Pellets oder Mikrokapseln enthält.

4. Formulierung gemäß einem der Ansprüche 1 bis 3,
**dadurch gekennzeichnet**, daß sie aus Buspiron oder einem Salz davon besteht, welches mit einem Adjuvans vermischt und/oder überzogen ist, welches ausgewählt ist unter einem synthetischen Polymer vom Polyvinyl-Typ, vom Polyethylen-Typ oder vom Cellulose-Typ, Fetten, Wachsen und Zuckern oder Kombinationen davon.

5. Formulierung gemäß einem der Ansprüche 1 bis 4 in Form einer Tablette, die Buspironhydrochlorid, welches eingebettet ist in Polyvinylchlorid und in Polyvinylacetat, und Magnesiumstearat als Gleitmittel enthält.

6. Formulierung gemäß einem der Ansprüche 1 bis 4 in Form einer Tablette, die Buspironhydrochlorid, eingebettet in Polyvinylchlorid und Ethylcellulose sowie, zusätzlich, in Hydroxypropylmethylcellulose, Natriumcarboxymethylcellulose oder Paraffin, und Magnesiumstearat als Gleitmittel enthält.

7. Formulierung gemäß einem der Ansprüche 1 bis 3 in Form einer Hartgelatinekapsel, die gefüllt ist mit Mikrokapseln oder beschichteten Pellets oder mit beschichteten oder unbeschichteten Mikropellets, welche Buspiron oder ein Salz davon enthalten.

8. Formulierung gemäß Anspruch 7 in Form einer Hartgelatinekapsel, die gefüllt ist mit beschichteten Mikrokügelchen, welche Buspironhydrochlorid, vermischt mit einem Zucker- und/oder einem Cellulosederivat, enthalten.

9. Formulierung gemäß einem der Ansprüche 1 bis 3 in Form einer Tablette, die Mikrokapseln oder beschichtete oder unbeschichtete Mikropellets umfaßt.

10. Verfahren zur Herstellung einer oralen Formulierung gemäß einem der Ansprüche 1 bis 9, dadurch gekennzeichnet, daß Buspiron oder ein Salz davon in einen Träger inkorporiert wird, der Buspiron oder ein Salz davon in einer in vitro Gesamtauflösungszeit von 6 bis 24 Stunden, die gemäß der USP XXI Paddle-Methode bei 50 bis 100 UpM bestimmt wird, zu wenigstens 80 % des Buspirongehaltes freisetzt.

11. Verfahren gemäß Anspruch 10,
**dadurch gekennzeichnet,** daß Buspiron oder ein Salz davon mit einem Adjuvans, welches ausgewählt ist unter einem synthetischen Polymer vom Polyvinyl-Typ, Polyethylen-Typ oder Cellulose-Typ, Fetten, Wachsen und Zuckern oder Kombinationen davon vermischt und/oder beschichtet wird und dann entweder zu Tabletten komprimiert wird oder zu Mikrokapseln/Pellets verarbeitet wird, die entweder in Hartgelatinekapseln gefüllt oder zu Tabletten komprimiert werden.

## Patentansprüche (Patentansprüche für folgende(n) Vertragsstaat(en): ES, GR)

1. Verfahren zur Herstellung einer oralen Formulierung zur kontrollierten/verzögerten Freisetzung von Buspiron oder einem Salz davon, wobei die in vitro Gesamtauflösungszeit für wenigstens 80 % des Buspirongehaltes zwischen 6 bis 24 Stunden beträgt, dadurch gekennzeichnet, daß Buspiron oder ein Salz davon in einen Träger inkorporiert wird, der Buspiron oder ein Salz davon in besagter in vitro Auflösungszeit freisetzt.

2. Verfahren gemäß Anspruch 1, das die Herstellung einer Formulierung umfaßt, welche 1 bis 80 % Gew./Gew. Buspiron oder eines Salzes davon, enthält.

3. Verfahren gemäß Anspruch 1 oder 2, das die Herstellung einer Matrixtablette oder einer Formulierung mit mehreren Kompartimenten, insbesondere einer Pellets oder Mikrokapseln enthaltenden Kapsel oder Tablette, umfaßt.

4. Verfahren gemäß einem der Ansprüche 1 bis 3, das die Herstellung einer Formulierung umfaßt, die aus Buspiron oder einem Salz davon besteht, welches mit einem Adjuvans, das ausgewählt ist unter einem synthetischen Polymer vom Polyvinyl-Typ, Polyethylen-Typ oder Cellulose-Typ, Fetten, Wachsen und Zuckern oder Kombinationen davon, vermischt und/oder beschichtet wird und dann entweder zu Tabletten komprimiert wird oder zu Mikrokapseln/Pellets verarbeitet wird, die entweder in Hartgelatinekapseln gefüllt oder zu Tabletten komprimiert werden.

5. Verfahren gemäß einem der Ansprüche 1 bis 4, das die Herstellung einer Tablette umfaßt, die Buspironhydrochlorid, welches eingebettet ist in Polyvinylchlorid und Polyvinylacetat, und Magnesiumstearat als Gleitmittel enthält.

6. Verfahren gemäß einem der Ansprüche 1 bis 4, das die Herstellung einer Tablette umfaßt, die Buspironhydrochlorid, eingebettet in Polyvinylchlorid und Ethylcellulose sowie, zusätzlich, in Hydroxypropylmethylcellulose, Natriumcarboxymethylcellulose oder Paraffin, und Magnesiumstearat als Gleitmittel enthält.

7. Verfahren gemäß einem der Ansprüche 1 bis 3, das die Herstellung einer Hartgelatinekapsel, die gefüllt ist mit Mikrokapseln oder beschichteten Pellets oder mit beschichteten bzw. unbeschichteten Mikropellets, welche Buspiron oder ein Salz davon enthalten, umfaßt.

8. Verfahren gemäß Anspruch 7, das die Herstellung einer Hartgelatinekapsel umfaßt, die gefüllt ist mit beschichteten Mikrokügelchen, welche Buspironhydrochlorid, vermischt mit einem Zucker- und/oder einem Cellulosederivat, enthalten, wobei unbeschichtete Mikrokügelchen gebildet werden, die dann beschichtet und in die Kapseln gefüllt werden.

9. Verfahren gemäß einem der Ansprüche 1 bis 3, das die Herstellung einer Tablette umfaßt, die Mikrokapseln oder beschichtete bzw. unbeschichtete Mikropellets umfaßt.

10. Verfahren gemäß Anspruch 4, wobei die Formulierung entweder zu Tabletten komprimiert wird oder zu Mikrokapseln/Pellets verarbeitet wird, die entweder in Hartgelatinekapseln gefüllt werden oder zu Tabletten komprimiert werden.

## Revendications (Revendications pour l'(les) Etat(s) contractant(s) suivant(s): AT, BE, CH, DE, FR, GB, IT, LI, LU, NL, SE)

1. Formule orale pour libération contrôlée/prolongée de buspirone ou son sel, caractérisée en ce que le temps total de dissolution in vitro, déterminé selon la méthode de la palette USP XXI à 50 OU 100 t/mn, est compris entre 6 et 24 heures pour au moins 80 pour cent de la teneur en buspirone.

2. Formule selon la revendication 1, caractérisée en ce qu'elle contient 1 à 80 pour cent en poids/poids de buspirone ou son sel.

3. Formule selon la revendication 1 ou 2, sous la forme d'une formule en comprimé en matrice ou multicompartiment, en particulier une capsule ou un comprimé contenant des boulettes ou des microcapsules.

4. Formule selon l'une quelconque des revendications 1 à 3, caractérisée en ce qu'elle se compose de buspirone ou son sel mélangé et/ou enrobé d'un adjuvant sélectionné dans le groupe consistant en un polymère synthétique du type polyvinyle, du type polyéthylène ou du type cellulose, des matières grasses, des cires et des sucres ou leurs associations.

5. Formule selon l'une quelconque des revendications 1 à 4, sous la forme d'un comprimé contenant du chlorhydrate de buspirone noyé dans le chlorure de polyvinyle et l'acétate de polyvinyle et du stéarate de magnésium comme lubrifiant.

6. Formule selon l'une quelconque des revendications 1 à 4, sous la forme d'un comprimé contenant du chlorure de buspirone noyé dans le chlorure de polyvinyle et de l'éthyl cellulose et, additionnellement, de l'hydroxypropyl méthyl cellulose, de la carboxyméthyl cellulose de sodium ou de la paraffine ainsi que du stéarate de magnésium comme lubrifiant.

7. Formule selon l'une quelconque des revendications 1 à 3, sous la forme d'une capsule en gélatine dure remplie de microcapsules ou de boulettes enrobées ou bien de microboulettes enrobées ou non enrobées contenant la buspirone ou son sel.

8. Formule selon la revendication 7, sous la forme d'une capsule en gélatine dure remplie de microsphères enrobées contenant du chlorhydrate de buspirone en mélange avec un sucre et/ou un dérivé de cellulose.

9. Formule selon l'une quelconque des revendications 1 à 3, sous la forme d'un comprimé comprenant des microcapsules ou des microboulettes enrobées ou non enrobées.

10. Procédé de préparations d'une formule orale selon l'une quelconque des revendications 1 à 9, caractérisé en ce que la buspirone ou son sel est incorporé dans un support qui libère la buspirone ou son sel en un temps total de dissolution in vitro,déterminé selon la méthode de la palette USP XXI à 50 ou 100 t/mn, entre 6 et 24 heures pour au moins 80 pour cent de la teneur en buspirone.

11. Procédé selon la revendication 10, caractérisé en ce que la buspirone ou son sel est mélangé et/ou enrobé d'un adjuvant choisi dans le groupe consistant en un polymère synthétique du type polyvinyle, du type polyéthylène ou du type cellulose, des matières grasses, cires et sucres ou leurs associations puis est soit compressé en comprimés ou traité en microcapsules/boulettes qui sont introduites dans des capsules en gélatine dure ou compressées en comprimés.

## Revendications (Revendications pour l'(les) Etat(s) contractant(s) suivant(s): ES, GR)

1. Procédé pour la préparation d'une formule orale pour une libération contrôlée/prolongée de buspirone ou son sel ayant un temps total de dissolution in vitro compris entre 6 et 24 heures pour au moins 80 pour cent de la teneur en buspirone, caractérisé en ce que la buspirone ou son sel est incorporé dans un support qui libère la buspirone ou son sel dans ledit temps de dissolution in vitro.

2. Procédé selon la revendication 1, qui comprend la préparation d'une formule contenant 1 à 80% en poids/poids de buspirone ou son sel.

3. Procédé selon la revendication 1 ou 2, qui comprend la préparation d'une formule en comprimé en matrice ou multicompartiment, en particulier une capsule ou un comprimé contenant des boulettes ou des microcapsules.

4. Procédé selon l'une quelconque des revendications 1 à 3, qui comprend la préparation d'une formule consistant en buspirone ou son sel mélangé et/ou enrobé d'un adjuvant sélectionné dans le groupe consistant en un polymère synthétique du type polyvinyle, du type polyéthylène ou du type cellulose, des matières grasses, des cires et des sucres ou leurs associations puis est soit compressé en comprimés ou traité en microcapsules/boulettes qui sont introduites dans des capsules en gélatine dure ou compressées en comprimés.

5. Procédé selon l'une quelconque des revendications 1 à 4, qui comprend la préparation d'un comprimé contenant du chlorhydrate de buspirone noyé dans le chlorure de polyvinyle et de l'acétate de polyvinyle et avec du stéarate de magnésium comme lubrifiant.

6. Procédé selon l'une quelconque des revendications 1 à 4, qui comprend la préparation d'un comprimé contenant du chlorhydrate de buspirone noyé dans le chlorure de polyvinyle et de l'acétate de cellulose,additionnellement de l'hydroxypropyl méthyl cellulose, de la carboxyméthyl cellulose de sodium ou de la paraffine ainsi que du stéarate de magnésium comme lubrifiant.

7. Procédé selon l'une quelconque des revendications 1 à 3, qui comprend la préparation d'une capsule en gélatine dure remplie de microcapsules ou de boulettes enrobées ou de microboulettes enrobées ou non enrobées contenant de la buspirone ou son sel.

8. Procédé selon la revendication 7, qui comprend la préparation d'une capsule en gélatine dure contenant du chlorhydrate de buspirone en mélange avec un sucre et/ou un dérivé de cellulose pour former des microsphères non enrobées qui sont alors recouvertes et introduites dans des capsules.

9. Procédé selon l'une quelconque des revendications 1 à 3, qui comprend la préparation d'un comprimé comprenant des microcapsules ou des microboulettes enrobées ou non enrobées.

10. Procédé selon la revendication 4, où la formule est soit compressée en comprimés ou traitée en microcapsules/boulettes qui sont soit introduites dans des capsules en gélatine dure ou compressées en comprimés.
